# EUROPÄISCHE PATENTANMELDUNG

(11) **EP 1 886 663 A2**
(43) Veröffentlichungstag der Anmeldung: **13.02.2008**
(21) Anmeldenummer: 07110600.9
(22) Anmeldetag: 19.06.2007
(51) Int. Cl.: A61K 8/44, A61K 8/46, A61K 8/73, A61K 8/81, A61K 8/86, A61Q 5/02, A61Q 5/12

(54) **Haarreinigungszusammensetzung mit besonderer Schaumleistung**

(30) Priorität: 28.06.2006 DE 102006030091
(71) Anmelder: Beiersdorf Aktiengesellschaft, 20253 Hamburg (DE)
(72) Erfinder: Albrecht, Harald, 8320, Fehraltorf (CH); Neuhoff, Henrike, 22359, Hamburg (DE); Wilken, Maren, 22848, Norderstedt (DE); Heitmann, Birgit, 22769, Hamburg (DE)
(74) Vertreter: Porath, Stefan

(57) **Zusammenfassung**

Kosmetische waschaktive Zubereitungen zur Reinigung der Haut und/oder zur Haar- und Kopfhautreinigung, enthaltend nichtionische, hochmolekulare Polyethylenglykole, Tenside und kationische Pflegestoffe, dadurch gekennzeichnet, dass der Schaumparameter Cremigkeit /Reichhaltigkeit den Wert 8 bis 10 aufweist.

## Beschreibung

Die vorliegende Erfindung betrifft kosmetische Reinigungsmittel mit besonderer Schaumleistung. Kosmetische Reinigungsmittel sind an sich bekannt. Es handelt sich dabei im Wesentlichen um oberflächenaktive Substanzen oder Stoffgemische, die dem Verbraucher in verschiedenen Zubereitungen angeboten werden.

Der Schaum eines Haarreinigungproduktes spielt bei der Anwendung für den Verbraucher eine große Rolle. Unter Schaumleistung versteht man das Aufschäumvermögen, die Menge an Schaum, die während der Applikation generiert wird und vor allem die Cremigkeit / Reichhaltigkeit des Schaums. Ein feinporiger, cremiger und reichhaltiger Schaum wird von den Verbrauchern als ideal angesehen.

Unter Pflegeleistung im Sinne der vorliegenden Schrift versteht man das Vermögen der Zusammensetzung beim Benutzer derselben sauberes Haar, eine gute Kämmbarkeit des Haares, im nassen und trockenen Zustand, eine gute Frisierbarkeit, einen guten Griff und einen guten Haarglanz zu erzielen.

Zubereitungen dieser Art sind beispielsweise Schaum- und Duschbäder, Shampoos, spezielle Reinigungsmittel für Kleinkinder und Babys und dergleichen.

Moderne Haarreinigungsprodukte bestehen im Allgemeinen aus Wasser, Tensiden, Verdicker, Hilfsstoffen und Pflegekomponenten.

Besonders den Pflegekomponenten kommt in neuerer Zeit eine hohe Bedeutung zu, da der Verbraucher, speziell einer mit strapaziertem/geschädigtem Haar, eine sehr hohe Pflegeleistung erwartet. Pflegende- bzw. konditionierende Substanzen sind Stoffe, die eine hohe Affinität zum Haar haben und auf die Haaroberfläche aufziehen wobei sie das Haar möglichst nicht beschweren. Sie glätten so die Haarstruktur und erleichtern dadurch das Kämmen der Haare. Gleichzeitig verhindern sie die elektrostatische Aufladung und verleihen dem Haar Glanz. Eine entscheidende Rolle bei den Konditionierstoffen kommt dabei auch der Sensorik - dem Gefühl und Griff den sie im nassen und trockenen Haar hervorrufen, zu. Pflege ist daher gleichbedeutend mit den biophysikalischen Parametern Kämmbarkeit, Volumen, Fülle, Glanz, Griff und Geschmeidigkeit.

Darüber hinaus beeinflusst die Konsistenz des Produkts, die Schaummenge und die Schaumcremigkeit, insbesondere zum Zeitpunkt der Schaumentwicklung, die Akzeptanz eines Produkts beim Verbraucher. Bereits bei der Produktverteilung und der Schaumentwicklung in der Hand oder in den Haaren bewertet der Verbraucher das Produkt hinsichtlich der zu erwartenden Pflegeleistung. Eine cremige, reichhaltige Schaumentwicklung ist hierbei gleichbedeutend mit der Erwartungshaltung der reichhaltigen Pflegeleistung. Dieses wird in der Regel durch eine Erhöhung der Konsistenz mit unterschiedlichen Verdickern erreicht. Es können so jedoch keine Shampoos mit optimalen Fließ- und Entnahmeeigenschaften bei gleichzeitig optimalen Pflegeeigenschaften hergestellt werden.

Pflegekomponenten werden generell in zwei Gruppen aufgeteilt: wasserlösliche und wasserunlösliche Konditionierstoffe. Zu den wasserunlöslichen Konditionierstoffen gehören Silikone, Öle, Fettalkohole und Wachse; zu den Wasserlöslichen kationische Tenside und Kationische Polymere.

Kationische Polymere sind für Shampoo-Formulierungen essentielle Rohstoffe. Zur Erzielung eines kontrollierten Konditioniereffektes gibt es mehrere Möglichkeiten. Dazu können besonders geeignete Polymere oder Tenside eingesetzt werden. Deren Einsatz beeinträchtigt oder beeinflusst aber fast immer die Schaumleistung des Produktes.

Wünschenswert ist es daher, ein Tensid/Polymersystem einzusetzen, dass hohe Pflegeeigenschaften aufweist und dem Produkt eine Ausreichende Schaumleistung verleiht.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Die Schrift WO 2000066081 offenbart die Kombination aus anionischen Tensiden, nichtflüchtigem Konditioniermittel, Partikel eines Antischuppenwirkstoffs, Polyquat und 0,005-1,5% PEG.

Die Schrift DE 69109973 offenbart Haarkosmetika, enthaltend 0,01-5% PEG mit einem Mw größer/gleich 500.000, 0,1-30% Öl oder Fett, 0,1-30% nichtionischem Tensid.

Die Schrift EP 485212 offenbart Reinigungszusammensetzung, enthaltend anionisches Tensid (>5%), Betain (>1%) und ein Hauterweichungsmittelmaterial (= Feuchthaltemittel) und oklusive Materialien (Öle, Wachse, Benetzungsmittel).

Die Schrift WO 2002087519 offenbart eine Rasierzubereitung bestehend auf PEG mit einem Molekumargewicht von 1 bis 5 Mda in Kombination mit natürlichen oder synthetischen Polysachariden (Gumms).

Die Schrift WO 2006/010441 A1 offenbart Hair Care Zusammensetzungen enthaltend PEGs mit MW größer/gleich 6 MDa.

Der Stand der Technik ließ es aber an Shampooformulierungen mangeln, welche dem geschädigten Haar in befriedigender Weise Pflege zukommen ließen. Und zugleich eine besondere Schaumleistung aufwiesen. Aufgabe war daher, auch diesen Nachteilen des Standes der Technik Abhilfe zu schaffen.

Es hat sich überraschend gezeigt, und darin liegt die Lösung dieser Aufgaben, dass kosmetische waschaktive Zubereitungen zur Reinigung der Haut und/oder zur Haar- und Kopfhautreinigung, enthaltend nichtionische, hochmolekulare Polyethylenglykole, Tenside und kationische Pflegestoffe, wobei der Schaumparameter Cremigkeit / Reichhaltigkeit den Wert 8 bis 10 aufweist, den Nachteilen des Standes der Technik abhelfen. Solche Zubereitungen stellen Shampoos dar, die sich beim Anschäumen besonders cremig und reichhaltig anfühlen und dabei optimale Haarpflegeeigenschaften aufweisen, ohne dabei gleichzeitig die Konsistenz zu erhöhen. Dadurch kommt es nicht zu den genannten Nachteilen des Fließverhaltens, wie bei dem Einsatz üblicher Verdicker oder zur Reduzierung der Pflegeeigenschaften, wie beim Einsatz von Polyacrylaten. Dabei ist es von Vorteil, wenn nichtionische, hochmolekulare Polyethylenglykole mit Molmassen im Bereich von 100.000 bis 6.000.000, bevorzugt mit einer Molmasse von 1.000.00 bis 5.000.000, besonders bevorzugt mit einer Molmasse von 4.000.000 eingesetzt werden, beispielsweise POLYOX WSR-301, bzw. POLYOX-WSR LEO NF von Amerchol. Dabei ist es von Vorteil, wenn das oder die Polyethylenglykole in Konzentrationen von 0,01-0,5 Gew %. bevorzugt 0,02-0,3 Gew.-% vorliegen. Dabei ist es von Vorteil, wenn das oder die Tenside anionische, amphotere und/oder nichtionische Tenside sind, die in Gesamtkonzentrationen von 2 bis 25 Gew.-% eingesetzt werden. Dabei ist es von Vorteil, wenn die Zubereitung zusätzlich Perlglanzmittel enthält, bevorzugt enthaltend eine Kombination von Laurylethersulfat mit Cocamidopropylbetain und Disodium PEG-5 Laurylcitrat Sulfosuccinat. Dabei ist es von Vorteil, wenn Laurylethersulfat in Konzentrationen von 5 -15 Gew.-% und Cocamidopropylbetain in Konzentration von 0,5 -10 Gew.-% und Disodium PEG-5 Laurylcitrat Sulfosuccinat in einer Konzentration von 0,5 - 5 Gew.-% enthalten ist, besonders bevorzugt liegen Laurylethersulfat in Konzentrationen von 6-12 Gew.-% und Cocamidopropylbetain in Konzentrationen von 1-5 Gew.-% und Disodium PEG- Laurylcitrat Sulfosuccinat in einer Konzentration von 1,5 - 4 Gew.-% vor. Dabei ist es von auch von besonderem Vorteil, wenn die Zubereitung klar und frei von Cocamidopropylbetain ist. Bei all dem ist es von Vorteil, wenn als Verdicker Natriumchlorid, PEG-200 hydrogeniertes Glycerylpalmitat, PEG-90 Glyceryl Isostearate oder Acrylat Copolymere verwendet werden, bevorzugt in Konzentrationen von 0.05 - 5 Gew.-%.

Dabei ist es von Vorteil, wenn zusätzlich Polyquaternium-10 oder Guar Quats oder Polyquaternium 7 oder eine Kombination aus Polyquaternium-10 und Guar Quat enthalten ist, bevorzugt entweder eine Kombination aus Guar Quat und Polyquaternium 7, oder eine Kombination aus Guar Quat und Polyquaternium 10, oder eine Kombination aus Polyquaternium-10 und Polyquaternium 7 enthalten ist, wobei gegebenenfalls der Gehalt an Polyquaternium-7 0,01 0,6 Gew.-% beträgt und gegebenenfalls der Gehalt an Polyquaternium-10 0,01-0,6 Gew.-% beträgt. Dabei ist es von Vorteil, wenn zusätzlich PEG-3 Distearat mit Teilchengrößen verwendet wird, die so bemessen sind, dass 90% der Volumenanteile eine Teilchengröße von kleiner 50µm aufweisen, bevorzugt beträgt der Gehalt an PEG-3 Distearat1,0 - 15 Gew.-%. Anstelle des Perlglanzsystems kann auch ein Trübungsmittel eingesetzt werden. Besonders vorteilhaft ist der Einsatz von Mischungen, bestehend aus Glycol Distearat, Coco-Glucosid, Glyceryl Oleat, Glyceryl Stearate wie sie unter dem Handelsnamen Lamesoft TM Benz (Cognis) angeboten weden. Vorteilhaft kann auch eine Kombination von Perlglanz und Trübungsmittel eingesetzt werden. Erfindungsgemäße Zubereitungen werden bevorzugt zur Reinigung der Haut und/oder zur Haar- und Kopfhautreinigung verwendet.

Anionische Tenside im Sinne der Erfindung weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische lonen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine Ionen.

### A. Anionische Tenside

Erfindungsgemäß vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

### Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat, Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
sowie
Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Amphotere Tenside

Erfindungsgemäß vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

Vorteilhafte nichtionische Tenside mit einem HLB-Wert von 10 bis 20 können beispielsweise sein:
PEG-20 Mandelölglyzerid, PEG-20 Nachtkerzenölglyzerid, PEG-60 hydrogeniertes Rizinusöl, Polysorbat 60, PEG-20 Sorbitan Isostearat, PEG-40 hydrogeniertes Rizinusöl, Polysorbat 80, Succrose Cocoat, PEG-45 Palmkernölglyzeride, PEG-45 Distelölglyzerid, PEG-60 Nachtkerzenölglyzerid, PEG-42 Babassuölglyzerid, PEG-20 Rizinusoleat, Steareth-21, Oleth-20, Polysorbat 40, Laureth-9, Laureth-15, Ceteareth-20, Ceteth-20, PEG-12 Laurat, PEG-24 Stearat, PEG-20 Stearat, PEG-20 Oleat, PEG-75 Lanolin, Laneth-40, PEG-8 Dilaurat, Polysorbat 65, PEG-7 Glyceryl Cocoat, Laneth-10, PEG-12 Oleat, Polyglyzeryl-6 Laurat, Polyglyzeryl-10 Laurat, Polyglyzeryl-10 Myristat, Polyglyzeryl-10 Stearat, Polyglyzeryl-10 Oleat, Polyglyzeryl-10 Isostearat, Polyglyzeryl-10 Diisostearat, PEG-15 Glyzeryloleat, PEG-60 Sorbitan Tetraoleat, PEG-10 Oleylether

Die erfindungsgemäßen waschaktiven Zubereitungen zeichnen sich in der Regel durch einen Wassergehalt von 95 - 5 Gew.-% aus, bezogen auf das Gesamtgewicht der Zubereitungen und stellen Gele dar.

Zubereitungen gemäß der Erfindung sind vorteilhaft auf einen pH-Bereich > 4,2 gepuffert, besonders bevorzugt > 4,5 besonders bevorzugt 4,8-7,5.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen (Filmbildner). Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen in Shampoos stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Erfindungsgemäß vorteilhafte Filmbildner stellen Cellulosederivate, quaternisierte Guar Gum Derivate und Dimethyldiallylammoniumchlorid-Acrylamid Copolymere dar.

Bei den kationischen Hydroxyethylcellulosederivaten haben sich Polyquaternium 10 Typen mit einem Molekulargewicht von 400.000 g/mol und einer mittleren Ladungsdichte von 1,07 - 1,57 als geeignet gezeigt. Als geeignet haben sich auch Polyquarternium-10 mit einem Molekulargewicht von 250.000 g/mol und einer höheren Ladungsdichte von 1,7meg/g gezeigt.

Bei den quaternisierte Guar Gum Derivate haben sich insbesondere Guar Hydroxypropyltrimoniumchlorid (z.B. Jaguar Excel®, Jaguar C 162® der Firma Rhodia, CAS 65497-29-2, CAS 39421-75-5) dar. Das Molekulargewicht dieser Derivate liegt typischer Weise in einem Bereich von 50.000 bis 2.500.000. Als besonders geeignet haben sich Molekulargewichte von 1.000.000 bis 1.500.000 gezeigt. Insbesonders geeinet ist Guar Hydroxypropyltrimonium Chlorid mit einem Molekulargewicht von 1.300000 und einer Ladungsdichte von 0.4-0.6 meq/g.

Als Konditioniermittel besonders geeignet haben sich Dimethyldiallylammoniumchlorid-Acrylamid Copolymere mit einem Molekulargewicht von 1,4x10⁶-1,8x10⁶ g/mol und einer Ladungsdichte von 2,5 - 3,5 gezeigt.

Als sehr gut hat sich auch die Kombination von Dimethyldiallylammoniumchlorid-Acrylamid Copolymere mit kationischen Hydroxyethylcellulosederivaten gezeigt. Bei der Kombination von Dimethyldiallylammoniumchlorid-Acrylamid Copolymer und Polyquaternium-10 haben sich Einsatzkonzentrationen von jeweils 0,01 - 1 % als vorteilhalft gezeigt. Besonders geeignet waren Einsatzkonzentrationen von 0,01 - 0,5 %.

Bei der Kombination von Dimethyldiallylammoniumchlorid-Acrylamid Copolymer und Guar Hydroxypropyltrimonium Chlorid haben sich Einsatzkonzentrationen von jeweils 0,01 - 1 % als vorteilhaft gezeigt. Besonders geeignet waren Einsatzkonzentrationen von 0,01 - 0,5 %.

Die Herstellung der erfindungsgemäßen kosmetischen und/oder dermatologischen Zubereitungen erfolgt in der dem Fachmann üblichen Weise, zumeist dergestalt, dass die erfindungsgemäß verwendeten grenzflächenaktiven Stoffe bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wässrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen lässt. Nach Abkühlen auf Raumtemperatur kann, insbesondere, wenn noch flüchtige Bestandteile eingearbeitet werden sollen, nochmaliges Homogenisieren erfolgen.

Die Bewertung der Cremigkeit / Reichhaltigkeit erfolgt duch ein Expertenpanel mit einem Standard Waschverfahren. Das Panel besteht aus 10-15 Personen trainierten Personen.

Standard Waschverfahren: Die Haarsträhnen werden zuerst gut mit warmen Wasser (38°C) durchfeuchtet und dann in eine Halterung fest eingespannt. Es werden 1,8 ml des Produktes auf die Finger gegeben. Das Produkt wird mittels zwei Streichbewegungen zwischen den Fingern beider Hände verteilt. Anschliessend wird das Shampoo mit drei Streichbewegungen von oben nach unten auf einer Haarsträhne verteilt. Mittels kreisender Bewegungen entlang der Haarstähne (von oben nach unten) wird das Shampoo mit beiden Händen auf der Strähne angeschäumt. Dabei machen die Hände immer abwechselnd kreisende Bewegungen auf der Haarsträhne, während die jeweils andere Hand die Strähne am unteren Ende festhält. Nach 30 Sekunden wird die Cremigkeit/Reichhaltigkeit des angeschäumten Shampoos auf der Haarsträhne anhand einer 10er Skala beurteilt. Dabei bedeutet 0 = wässriger Schaum (reines Wasser) und 10 = cremig / reichhaltiger Schaum (idealer Schaumwert, der bislang von keinem auf dem Markt befindlichen Produkt erreicht wird). Derzeitige auf dem Markt erhältliche Shampoos erreichen auf dieser Skala Werte zwischen 3 und 6. Die beschriebene Neuentwicklung (Rezeptur 1) erreicht einen Wert des Schaumparameters Cremigkeit / Reichhaltigkeit von 8.

Zusätzlich wird die Schaumqualität, als prozentualer Anteil von feinblasigem, mittelblasigem und grobblasigem Schaum nach dem Anschäumen ermittelt.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

In einem Phasenkessel wird jeweils die Hauptmenge Wasser vorgelegt, anschließend werden die wasserlöslichen Bestandteile (Polyethylenglykole), die Tenside, die Konservierungsmittel und sonstige wasserlösliche Hilfsstoffe nacheinander zugegeben und miteinander vermischt, bis die Phase homogen ist.

**Perlglänzendes Shampoo:**

| | 1 | 2 | 3 | 4 | 5 | 6 | 7 | 8 |
|---|---|---|---|---|---|---|---|---|
| Natrium Laurylethersulfat | 9 | 5 | 7 | 9 | 9 | 10 | 12 | 15 |
| Cocamidopropyl Betain | 2 | 5 | 4 | 3,5 | 5 | 3 | 3 | 5 |
| Di-Natrium PEG-5 Laurylcitrat Sulfosuccinat | 3,5 | - | 4 | 3 | 3 | 2 | - | 4 |
| Decyl Glucoside | - | 3 | - | - | - | - | 3 | - |
| Polyquaternium-10 | - | 0,3 | 0,2 | 0,3 | 0,1 | 0,1 | 0,4 | 0,2 |
| Polyquaternium-7 | 0,3 | - | 0,3 | - | | 0,2 | | 0,5 |
| PEG-200 Hydrogenated Glyceryl Palmate | - | 0,8 | 1 | - | 1 | 0,5 | - | 1,5 |
| Guar Hydroxypropyltrimoniumchlorid | 0,1 | 0,1 | - | 0,1 | 0,1 | - | - | - |
| PEG-90M | 0,3 | 0,05 | 0,2 | 0,1 | 0,05 | 0,1 | 0,2 | 0,02 |
| PEG-40 hydrogeniertes Rizinusöl | 0,5 | 0,8 | 0,6 | 0,6 | 0,9 | 0,6 | 0,5 | 0,7 |
| PEG-3 Distearat | - | 2 | - | 1,5 | 1 | - | - | 3 |
| Glycol Distearat | 3 | - | 1,5 | - | - | 2 | 1 | - |
| PEG-7 Glycerylcocoat | 1,5 | - | - | - | - | - | - | 2 |
| Trisodium EDTA | - | 0,5 | - | - | 0,7 | - | - | - |
| Tetrasodium Iminodisuccinat | - | - | 0,7 | - | - | 0,5 | - | - |
| Natronlauge | q.s. | q.s. | q.s. | q.s. | q.s. | q.s. | q.s. | q.s. |
| Natrium Salicylat | 0,2 | 0,4 | 0,2 | 0,2 | 0,4 | 0,2 | 0,2 | 0,4 |
| Natrium Benzoat | 0,45 | 0,4 | 0,4 | 0,4 | 0,4 | 0,4 | 0,45 | 0,4 |
| Parfüm | q.s. | q.s. | q.s. | q.s. | q.s. | q.s. | q.s. | q.s. |
| Benzophenon-4 | - | - | - | - | 0,25 | - | 0,1 | - |
| Oryzanol | - | - | - | 0,1 | - | 0,05 | - | - |
| Lotusblütenextrakt | 0,1 | - | - | - | - | - | 0,05 | - |
| Citronensäure | q.s. | q.s. | q.s. | q.s. | q.s. | q.s. | q.s. | q.s. |
| Natriumchlorid | q.s. | q.s. | q.s. | q.s. | q.s. | q.s. | q.s. | q.s. |
| Avocodoöl | 0,3 | - | - | - | - | - | - | 0,5 |
| Wasser | ad 100 | ad 100 | ad 100 | ad 100 | ad 100 | ad 100 | ad 100 | ad 100 |

**Klares Shampoo:**

| | 1 | 2 | 3 | 4 | 5 | 6 | 7 |
|---|---|---|---|---|---|---|---|
| Natrium Laurylethersulfat | 10 | 7 | 5 | 9 | 12 | 15 | 9 |
| Di-Natrium PEG-5 Laurylcitrat Sulfosuccinat | 3 | - | 4 | 3 | 3 | 2 | - |
| Decyl Glucoside | - | 2 | 3 | - | - | - | 3 |
| Polyquaternium-10 | - | 0,2 | - | 0,3 | - | 0,5 | 0,2 |
| Polyquaternium-7 | 0,3 | - | 0,3 | - | - | 0,2 | - |
| PEG-200 Hydrogenated Glyceryl Palmate | 1 | 0,5 | 1,5 | 1,5 | 0,5 | 1 | 1,5 |
| Guar Hydroxypropyltrimoniumchlorid | 0,1 | - | 0,2 | 0,1 | 0,3 | - | - |
| PEG-90M | 0,1 | 0,2 | 0,05 | 0,1 | 0,2 | 0,05 | 0,3 |
| PEG-40 hydrogeniertes Rizinusöl | 0,5 | 0,7 | 0,9 | 0,5 | 0,6 | 0,8 | 0,5 |
| PEG-7 Glycerylcocoat | 2 | - | - | - | - | 2 | - |
| Trisodium EDTA | 0,7 | - | - | - | - | - | 0,5 |
| Tetrasodium Iminodisuccinat | - | - | 0,5 | - | - | 0,7 | - |
| Natronlauge | q.s. | q.s. | q.s. | q.s. | q.s. | q.s. | q.s. |
| Natrium Salicylat | - | - | 0,2 | - | - | - | 0,2 |
| Natrium Benzoat | 0,4 | 0,45 | 0,4 | 0,4 | 0,4 | 0,45 | 0,4 |
| Parfüm | q.s. | q.s. | q.s. | q.s. | q.s. | q.s. | q.s. |
| Benzophenon-4 | - | 0,25 | - | - | 0,1 | - | - |
| Oryzanol | 0,05 | - | - | 0,1 | - | - | - |
| Kamillenextrakt | - | - | 0,05 | - | - | - | 0,03 |
| Citronensäure | q.s. | q.s. | q.s. | q.s. | q.s. | q.s. | q.s. |
| Avocodoöl | 1 | - | - | - | - | 0.5 | - |
| Natriumchlorid | q.s. | q.s. | q.s. | q.s. | q.s. | q.s. | q.s. |
| Wasser | ad 100 | ad 100 | ad 100 | ad 100 | ad 100 | ad 100 | ad 100 |

**Mildes Shampoo**

| | 1 | 2 | 3 | 4 | 5 | 6 |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyceryl Palmate | - | - | - | 0.2 | 0.3 | 0,4 |
| Polyquaternium-7 | 0.3 | 0.1 | 0.2 | 0.3 | 0,4 | 0,5 |
| Polyquaternium-10 | 0,2 | 0.3 | 0,1 | 0,2 | 0.2 | 0,1 |
| PEG-3 Distearat | 1 | 0,75 | 0,5 | 1,5 | - | - |
| Glycol Distearat | - | - | - | - | 0.5 | 0,5 |
| PEG-90M | 0,05 | 0,75 | 0,1 | 0,05 | 0,1 | 0,05 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische waschaktive Zubereitungen zur Reinigung der Haut und/oder zur Haar- und Kopfhautreinigung, enthaltend nichtionische, hochmolekulare Polyethylenglykole, Tenside und kationische Pflegestoffe, **dadurch gekennzeichnet, dass** der Schaumparameter Cremigkeit / Reichhaltigkeit den Wert 8 bis 10 aufweist.

2. Zubereitung nach Patentanspruch 1 **dadurch gekennzeichnet, dass** nichtionische, hochmolekulare Polyethylenglykole mit Molmassen im Bereich von 100.000 bis 6.000.000, bevorzugt mit einer Molmasse von 1.000.00 bis 5.000.000, besonders bevorzugt mit einer Molmasse von 4.000.000 eingesetzt werden.

3. Zubereitung nach einem der vorangehenden Patentansprüche **dadurch gekennzeichnet, dass** das oder die Polyethylenglykole in Konzentrationen von 0,01-0,5 Gew %. bevorzugt 0,02-0,3 Gew.-% vorliegen.

4. Zubereitung nach einem der vorangehenden Patentansprüche **dadurch gekennzeichnet, dass** das oder die Tenside anionische, amphotere und/oder nichtionische Tenside sind, die in Gesamtkonzentrationen von 2 bis 25 Gew.-% eingesetzt werden.

5. Zubereitung nach einem der vorangehenden Patentansprüche **dadurch gekennzeichnet, dass** sie zusätzlich Perlglanzmittel enthält, bevorzugt einhaltend eine Kombination von Laurylethersulfat mit Cocamidopropylbetain und Disodium PEG-5 Laurylcitrat Sulfosuccinat.

6. Zubereitung nach einem der vorangehenden Patentansprüche **dadurch gekennzeichnet, dass** Laurylethersulfat in Konzentrationen von 5-15 Gew.-% und Cocamidopropylbetain in Konzentration von 0,5 -10 Gew.-% und Disodium PEG-5 Laurylcitrat Sulfosuccinat in einer Konzentration von 0,5 - 5 Gew.-% enthalten ist, besonders bevorzugt liegen Laurylethersulfat in Konzentrationen von 6-12 Gew.-% und Cocamidopropylbetain in Konzentrationen von 1 - 5 Gew. % und Disodium PEG- Laurylcitrat Sulfosuccinat in einer Konzentration von 1,5 - 4 Gew.-% vor.

7. Zubereitung nach einem der vorangehenden Patentansprüche **dadurch gekennzeichnet, dass** sie klar und frei von Cocamidopropylbetain ist.

8. Zubereitung nach einem der vorangehenden Patentansprüche **dadurch gekennzeichnet, dass** als Verdicker Natriumchlorid, PEG-200 hydrogeniertes Glycerylpalmitat, PEG-90 Glyceryl Isostearate oder Acrylat Copolymere verwendet werden, bevorzugt in Konzentrationen von 0.05 - 5 Gew.-%.

9. Zubereitung nach einem der vorangehenden Patentansprüche **dadurch gekennzeichnet, dass** zusätzlich Polyquaternium-10 oder Guar Quats oder Polyquaternium 7, bevorzugt entweder eine Kombination aus Guar Quat und Polyquaternium 7, oder eine Kombination aus Guar Quat und Polyquaternium 10, oder eine Kombination aus Polyquaternium-10 und Polyquaternium 7 enthalten ist, wobei gegebenenfalls der Gehalt an Polyquaternium-7 0,01-0,6 Gew.-% beträgt und gegebenenfalls der Gehalt an Polyquaternium-10 0,01 - 0,6 Gew.-% beträgt.

10. Zubereitungen nach einem der vorhergehenden Ansprüche, **dadurch gekennzeichnet, dass** zusätzlich PEG-3 Distearat mit Teilchengrößen verwendet wird, die so bemessen sind, dass 90% der Volumenanteile eine Teilchengröße von kleiner 50µm aufweisen, bevorzugt beträgt der Gehalt an PEG-3 Distearat1,0 -15 Gew.-%.
